# EUROPÄISCHE PATENTSCHRIFT

(11) **EP 1 536 757 B1**
(45) Veröffentlichungstag und Bekanntmachung des Hinweises auf die Patenterteilung: **23.10.2013**
(21) Anmeldenummer: 03773618.8
(22) Anmeldetag: 04.09.2003
(51) Int. Cl.: A61K 8/49, A61Q 5/10, A61K 8/67, A61K 8/60, A61K 8/34

(54) **HAARBEHANDLUNGSMITTEL**
HAIR TREATMENT AGENT
AGENT DE TRAITEMENT CAPILLAIRE

(30) Priorität: 13.09.2002 DE 10242748
(43) Veröffentlichungstag der Anmeldung: 08.06.2005
(73) Patentinhaber: Henkel AG & Co. KGaA, 40589 Düsseldorf (DE)
(72) Erfinder: KLEEN, Astrid, 22763 Hamburg (DE); HÖFFKES, Horst, 40595 Düsseldorf (DE); HOLLENBERG, Detlef, 40699 Erkrath (DE); HOWORKA, Wilfried, 40597 Düsseldorf (DE)
(86) Internationale Anmeldenummer: PCT/EP2003/009811
(87) Internationale Veröffentlichungsnummer: WO 2004/026272

(56) Entgegenhaltungen:
- WO-A-00/07559
- WO-A-03/047539
- DE-A- 10 002 725
- DE-A- 10 014 632
- DE-A- 19 933 464

## Beschreibung

Die Erfindung betrifft ein Oxidationshaarfärbemittel, enthaltend eine Spezielle Wirkstoffkombinationen, ein Verfahren zur oxidativen Färbung von Haaren mit diesem Mittel sowie die Verwendung des Mittels zur oxidativen Haarfärbung.

Das menschliche Haar wird heutzutage in vielfältiger Weise mit haarkosmetischen Zubereitungen behandelt. Dazu gehören beispielsweise die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung und Nuancierung der Farbe des Kopfhaares eine herausragende Rolle. Aber gerade die Haarfärbeverfahren können das Haar zusätzlich zu schädlichen Umwelteinflüssen negativ auf der Oberfläche beeinflussen und in ihrer Struktur schädigen. Strukturell geschädigtes Haar weist vielfach ein sprödes, stumpfes und unattraktives Aussehen auf, was bei dem Verbraucher zu Unzufriedenheit führt.

Des weiteren sind eine Reihe von Farbstoffvorprodukten bekannt, die auf der Kopfhaut ein erhöhtes allergenes Potential aufweisen und beim Verbraucher zu allergischen Reaktionen führen können.

Auch weitere Komponenten, die üblicherweise in den oben genannten Haarbehandlungsmitteln zum Einsatz kommen, können allergische Reaktionen auf der Haut- und/oder der Kopfhaut hervorrufen und/oder das Haar strapazieren. Beispiele für solche Komponenten sind bestimmte Parfumöle, Konservierungsstoffe wie Parabene und Sulfite, bestimmte Tenside, Fettalkohole wie Wollwachs und Oxidationsfarbstoffvorprodukte (sogenannte Entwickler- und Kupplerkomponenten) wie beispielsweise p-Phenylendiamin oder p-Toluylendiamin, aber auch Oxidationsmittel wie Wasserstoffperoxid wirken auf der Haut irritativ.

In der Vergangenheit sind daher zahlreiche Versuche unternommen worden, der Schädigung der Haarstruktur sowie dem allergenen Potential einiger Haarbehandlungs- und -färbemittel entgegenzuwirken, indem restrukturierende Substanzen, bzw. Alternativstoffe zu den allergenen Komponenten entwickelt und eingesetzt wurden. Dabei kamen eine Vielzahl von Pflegekomponenten zum Einsatz, die gezielt bei der Nachbehandlung bereits geschädigter Haare Anwendung fanden. Eine andere Variante umfasst die gleichzeitige Behandlung der Haare mit einem Färbemittel und einer Pflegekomponente - beispielsweise sind Formulierungen bekannt, die in einem handelsüblichen Fixiermittel oder Färbemittel weiterhin eine oder mehrere Pflegekomponenten enthalten. Dadurch sollte sowohl die Haarschädigung während der Behandlung minimiert, als auch bereits geschädigtes Haar während der erneuten Behandlung so weit wie möglich geschont werden. So wurde in der DE-A1-196 17 569 vorgeschlagen, spezielle Aminosäuren als Pflegestoffe zu verwenden.

Die Firma Beiersdorf offenbart in einer Reihe von Anmeldungen, beispielsweise in der DE-A1-199 33 463 und DE-A1-199 33 464, Hautpflegemittel auf der Basis von Ectoin und Ectoinderivaten sowie deren Verwendung, vorwiegend in Sonnenschutzformulierungen für die menschliche Haut. Die fakultative Verwendung von Ectoin oder Ectoinderivaten bei der Behandlung oxidativ geschädigter Haare wird ebenfalls genannt.

In der EP 671 161 A1 werden Ectoin und Ectoinderivate in kosmetischen Zubereitungen zur Behandlung von gealterter und gereizter Haut offenbart. Die WO 00/07560 beansprucht die Verwendung von Ectoin und bestimmter Derivate des Ectoins in kosmetischen Formulierungen zum Schutz der menschlichen Haut vor Trockenheit und zu hohen Salzkonzentrationen.

Es wurde nun überraschenderweise gefunden, dass mit einer völlig neuartigen Wirkstoffkombination, umfassend mindestens ein heterocyclisches Carbonsäurederivat und mindestens eine weitere Pflegekomponente, geschädigtes Haar behandelt und in signifikanter und unerwarteter Weise restrukturiert werden kann.

Unter Restrukturierung im Sinne der Erfindung ist eine Verringerung der durch verschiedenartige Einflüsse entstandenen Schädigungen keratinischer Fasern zu verstehen. Hierbei stellt beispielsweise die Wiederherstellung der natürlichen Festigkeit eine wesentliche Rolle. Beispiele für schädigende Einflüsse sind Dauerwellbehandlungen, oxidative Färbungen oder Aufhellungen der Haare sowie häufiges Waschen, Fönen und Kämmen. Weiterhin können Schädigungen durch Umwelteinflüsse, wie beispielsweise UV-Licht, auftreten. Restrukturierte Fasern zeichnen sich zum Beispiel durch einen erhöhten Glanz, einen verbesserten Griff und durch eine leichtere Kämmbarkeit aus. Ferner lässt sich eine erfolgreiche Restrukturierung physikalisch als Schmelzpunkterhöhung im Vergleich zur geschädigten Faser nachweisen.

Ein Verfahren zum Nachweis der Restrukturierung, auf das in dieser Anmeldung ausdrücklich Bezug genommen wird, wurde beispielsweise in der WO 01/21139 offenbart.

Gegenstand der vorliegenden Erfindung ist daher die Verwendung einer Wirkstoffkombination, bestehend aus mindestens einer Verbindung der allgemeinen Formel (I) oder (II) 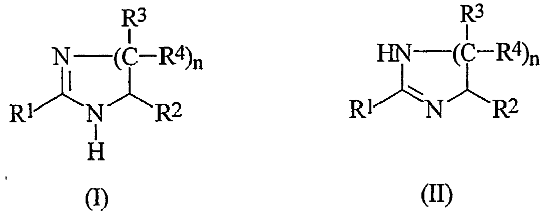 und/oder eines physiologisch verträglichen Salzes und/oder einer isomeren oder stereoisomeren Form dieser Verbindungen, in denen
- R¹ steht für ein Wasserstoffatom, einen verzweigten oder unverzweigten C₁-C₄-Alkylrest oder einen C₂-C₄-Hydroxyalkylrest,
- R² steht für ein Wasserstoffatom, eine Gruppierung -COOR⁵ oder eine Gruppierung -CO(NH)R⁵, wobei R⁵ für ein Wasserstoffatom, einen C₁-C₄-Alkylrest, einen Aminosäurerest, einen Dipeptid- oder einen Tripeptidrest stehen kann,
- R³ und R⁴ stehen unabhängig voneinander für ein Wasserstoffatom, einen C₁-C₄-Alkylrest oder einer der beiden Reste steht für eine Hydroxygruppe und
- n steht für eine ganze Zahl von 1 bis 3,
und mindestens einer weiteren Komponente, ausgewählt aus der Gruppe, die gebildet wird aus Alkylpolyglucosiden, Allantoin, sowie den physiologisch verträglichen Derivaten dieser Verbindungen, zur Behandlung von Haaren.

Geeignete physiologisch verträgliche Salze der allgemeinen Verbindungen gemäß der Formeln (I) oder (II) sind beispielsweise die Alkali-, Erdalkali-, Ammonium-, Triethylamin- oder Tris-(2-hydroxyethyl)aminsalze sowie solche, die sich aus der Umsetzung von Verbindungen der Formel (I) oder (II) mit anorganischen und organischen Säuren wie Salzsäure, Phosphorsäure, Schwefelsäure, verzweigten oder unverzweigten, substituierten oder unsubstituierten (beispielsweise durch eine oder mehrere Hydroxygruppen) C₁-C₄-Mono- oder Dicarbonsäuren, aromatische Carbonsäuren und Sulfonsäuren wie Essigsäure, Citronensäure, Benzoesäure, Maleinsäure, Fumarsäure, Weinsäure und p-Toluolsulfonsäure ergeben. Beispiele für besonders bevorzugte physiologisch verträgliche Salze sind die Na-, K-, Mg-, Ca- und Ammoniumsalze der Verbindungen gemäß der Formel (I) oder (II), sowie die Salze, die sich durch die Umsetzung von Verbindungen gemäß der Formel (I) oder (II) mit Salzsäure, Essigsäure, Citronensäure und Benzoesäure ergeben.

Unter isomeren und stereoisomeren Formen der Verbindungen gemäß Formel (I) oder (II) werden erfindungsgemäß alle auftretenden optischen Isomere, Diastereomere, Racemate, Zwitterionen, Kationen oder Gemische davon verstanden.

Unter dem Begriff Aminosäure werden die stereoisomeren Formen, z.B. D- und L-Formen, folgender Verbindungen verstanden: Asparagin, Arginin, Asparaginsäure, Glutamin, Glutaminsäure, β-Alanin, γ-Aminobutyrat, N_{ε}-Acetyllysin, N_{δ}-Acetylornitin, N_{γ}-Acetyldiaminobutyrat, N_{α}-Acetyldiaminobutyrat, Histidin, Isoleucin, Leucin, Methionin, Phenylalanin, Serin, Threonin und Tyrosin. L-Aminosäuren sind bevorzugt. Aminosäurereste leiten sich von den entsprechenden Aminosäuren ab. Die folgenden Aminosäuren sind bevorzugt:
Gly, Ala, Thr, Val, β-Ala, γ-Aminobutyrat, Asp, Glu, Asn, N_{ε}-Acetyllysin, N_{δ}-Acetylornitin, N_{γ}-Acetyldiaminobutyrat und N_{α}-Acetyldiaminobutyrat.

Die Kurzschreibweise der Aminosäuren erfolgte nach der allgemein üblichen Schreibweise. Die Di- oder Tripeptidreste sind ihrer chemischen Natur nach Säureamide und zerfallen bei der Hydrolyse in 2 oder 3 Aminosäuren. Die Aminosäuren in dem Di- oder Tripeptidrest sind durch Amidbindungen miteinander verbunden.

Bezüglich der Herstellung der Di- und Tripeptidreste wird ausdrücklich auf die EP 0 671 161 A1 der Firma Marbert verwiesen. Auch Beispiele für Di- und Tripeptidreste sind der Offenbarung der EP 0 671 161 A1 zu entnehmen.

Beispiele für C₁-C₄-Alkylgruppen in den erfindungsgemäßen Verbindungen sind Methyl, Ethyl, n-Propyl, Isopropyl, n-Butyl, Isobutyl und tert.-Butyl. Bevorzugte Alkylgruppen sind Methyl und Ethyl, Methyl ist eine besonders bevorzugte Alkylgruppe. Bevorzugte C₂-C₄-Hydroxyalkylgruppen sind die Gruppen 2-Hydroxyethyl, 3-Hydroxypropyl oder 4-Hydroxybutyl; 2-Hydroxyethyl ist eine besonders bevorzugte Hydroxylalkylgruppe.

Die erfindungsgemäß bevorzugten Vitamin- und Provitamin-Komponenten sind erfindungsgemäß Vitamin B3, Vitamin B6, Vitamin B12, Vitamin C, Vitamin E, Vitamin F, Vitamin H, Provitamin B5, Niacinamid und Pantolacton.

Insbesondere bevorzugte Vitamin- und Provitamin-Komponenten im Sinne der Erfindung sind Vitamin C, Vitamin B6, Vitamin H, Provitamin B5, Niacinamid und Pantolacton.

Weiterhin bevorzugt ist die Verwendung der erfindungsgemäßen Wirkstoffkombination in einem Gewichtsverhältnis der Verbindung nach Formel (I) oder (II) zu den Alkylpolyglucosiden von 1:1 bis 1:3 und/oder einem Gewichtsverhältnis der Verbindung nach Formel (I) oder (II) zu Allantoin von 2:1 bis 1:2.

Die Verwendung der Verbindungen gemäß Formel (I) oder (II), bei denen der Rest R¹ für eine Methylgruppe steht, ist ebenfalls bevorzugt.

Weiterhin ist die Verwendung derjenigen Verbindungen gemäß der allgemeinen Formel (I) oder (II) erfindungsgemäß begünstigt, bei denen der Rest R² für die Gruppierung-COOH steht.

Bevorzugt ist auch die Verwendung von Verbindungen der allgemeinen Formel (I) oder (II), bei denen die Reste R³ und R⁴ für Wasserstoff, oder jeweils unabhängig voneinander für Wasserstoff oder eine Hydroxygruppe stehen.

Ferner ist besonders die Verwendung solcher Verbindungen gemäß der allgemeinen Formel (I) oder (II) bevorzugt, bei denen n für die Zahl 2 steht.

Die Verwendung von (S)-2-Methyl-1,4,5,6-tetrahydro-4-pyrimidincarbonsäure (Ectoin) als Verbindung der allgemeinen Formel (I) oder (II) sowie die physikalisch verträglichen Salze dieser Verbindung, ist ganz besonders bevorzugt.

Ebenfalls ganz besonders bevorzugt ist die Verwendung von (S,S)-5-Hydroxy-2-methyl-1,4,5,6-tetrahydro-4-pyrimidincarbonsäure (Hydroxyectoin) als Verbindung der allgemeinen Formel (I) oder (II) sowie die physiologisch verträglichen Salze dieser Verbindung.

Die erfindungsgemäßen Wirkstoffkombinationen, insbesondere mit Ectoin oder dessen physiologisch verträglichen Salzen als Verbindung der allgemeinen Formel (I) oder (II), können erfindungsgemäß in allen Mitteln verwendet werden, die der Reinigung und Behandlung von Haaren dienen. Beispiele für solche Haarbehandlungsmittel sind Haarshampoos, Haarspülungen, Haarkuren, Haar-Tonics, Dauerwell- und oxidative Haarfärbemittel, Haarfärbeshampoos, Haarfestiger, Haarlegemittel, Haarstyling-Zubereitungen, z.B. Fönwell-Lotionen, Schaumfestiger, Haargele, Haarwachse und andere Haarreinigung- und Haarbehandlungsmittel.

Insbesondere bevorzugt ist die Verwendung der erfindungsgemäßen Wirkstoffkombination in oxidativen Haarfärbemitteln.

Generell können die Verbindungen gemäß der allgemeinen Formel (I) oder (II), insbesondere Ectoin oder dessen physiologisch verträgliche Salze, in den Haarreinigungs-, Haarbehandlungs- und Haarfärbemitteln in einem Konzentrationsbereich von 0,01 bis 5 Gew.-%, bezogen auf das Gesamtgewicht des Mittels, verwendet werden.

Insbesondere bevorzugt ist die Verwendung einer Einsatzmenge von 0,1 bis 1 Gew.-% der Verbindung nach Formel (I) oder (II), insbesondere von Ectoin oder dessen physiologisch verträglichen Salzen, bezogen auf das Gesamtgewicht des Mittels.

Die Verwendung von Wirkstoffkombinationen, bei denen die Vitaminkomponente Vitamin C oder eines der physiologisch verträglichen Derivate des Vitamins C ist, kann erfindungsgemäß bevorzugt sein.

Des weiteren ist die Verwendung derjenigen Wirkstoffkombinationen erfindungsgemäß bevorzugt, bei denen das Provitamin Panthenol (Provitamin B5) oder ein physiologisch verträgliches Derivat des Provitamins B5 ist.

Generell kann die Verwendung der Vitamin- und/oder die Provitamin-Komponente sowie deren physiologisch verträglichen Derivaten in den Haarreinigungs-, Haarbehandlungs- und Haarfärbemitteln in einem Konzentrationsbereich von 0,01 bis 5 Gew.-%, bezogen auf das Gesamtgewicht des Mittels, erfolgen.

Insbesondere bevorzugt ist eine Einsatzmenge von 0,1 bis 1 Gew.-% der Vitamin- und/oder die Provitamin-Komponente sowie deren physiologisch verträglichen Derivaten.

Erfindungsgemäß bevorzugt ist die Verwendung einer Wirkstoffkombination, die als Alkylpolyglucosid-Komponente für eine Verbindung der allgemeinen Formel R'O-(Z)ₓ steht, in der R' für einen Alkylrest mit 6 bis 22 Kohlenstoffatomen, Z einen Zuckerbaustein und x 1,1 bis 5 Zuckereinheiten bedeutet.

Insbesondere bevorzugt sind Alkylpolyglucoside mit einer C₁₂-C₁₆-Alkylkette, einem C₆-Zuckerbaustein und einem x-Wert von 1,4.

Bevorzugt ist die Verwendung einer Einsatzmenge von 0,1 bis 10 Gew.-% der Alkylpolyglucosid-Komponente, bezogen auf das Gesamtgewicht des Mittels.

Ein weiterer Gegenstand der Erfindung ist ein Oxidationshaarfärbemittel, das in einem zum Färben geeigneten Medium neben der erfindungsgemäßen Wirkstoffkombination mindestens ein Farbstoffvorprodukt, ausgewählt aus der Gruppe der Entwicklerkomponenten, enthält.

Als Oxidationsfarbstoffvorprodukte vom Entwickler-Typ werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrozone, 4-Aminopyrazolderivate sowie 2,4,5,6-Tertaaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (III) 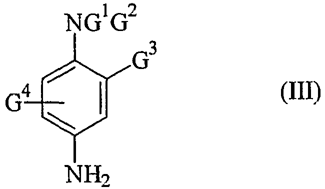 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁-bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁-bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, Iod-oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄-Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende a,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (III) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Di-methyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (III) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (IV) entsprechen, sowie ihre physiologisch verträglichen Salze: 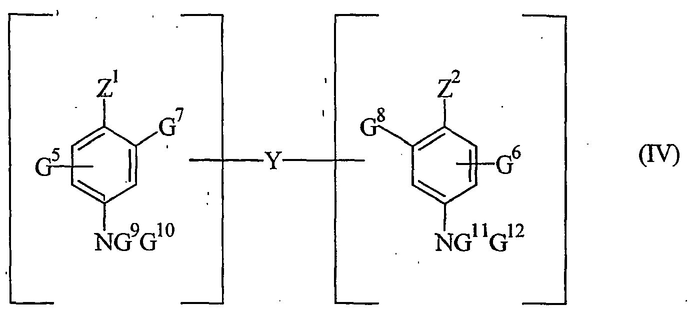 wobei:
- Z¹ und Z² stehen unabhängig voneinander für einen Hydroxyl- oder NH₂-Rest, der gegebenenfalls durch einen C₁- bis C₄-Alkylrest, durch einen C₁-bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄- Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest, mit den Maßgaben, daß
- die Verbindungen der Formel (IV) nur eine Verbrückung Y pro Molekül enthalten und
- die Verbindungen der Formel (IV) mindestens eine Aminogruppe enthalten, die mindestens ein Wasserstoffatom trägt.

Die in Formel (IV) verwendeten Substituenten sind erfindungsgemäß analog zu den obigen Ausführungen definiert.

Bevorzugte zweikernige Entwicklerkomponenten der Formel (IV) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piper-azin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (IV) sind N,N'-Bis-(β-hydroxyethyl)-N,N'-bis-(4'-aminophenyl)-1,3-diamino-propan-2-ol, Bis-(2-hydroxy-5-aminophenyl)-methan, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (V) 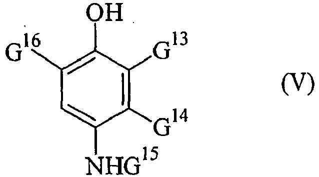 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁-bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁-bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁-bis C₄)-Alkoxy-(C₁- bis C₄)-alkylrest, einen C₁- bis C₄-Aminoalkylrest oder einen C₁-bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (V) verwendeten Substituenten sind erfindungsgemäß analog zu den obigen Ausführungen definiert.

Bevorzugte p-Aminophenole der Formel (V) sind insbesondere p-Aminophenol, N-Methyl-p-aminophenol, 4-Amino-3-methyl-phenol, 4-Amino-3-fluorphenol, 2-Hydroxy-methylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(ß-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (V) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)-amino-3-amino-pyridin, 2,3-Diamino-6-methoxy-pyridin, 2-(ß-Meth-oxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethyl-amino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(ß-hydroxyethyl)-pyrazol, 3,4-Diaminopyrazol, 4,5-Diamino-1-(4'-chlor-benzyl)-pyrazol, 4,5-Diamino-1,3-dimethylpyrazol, 4,5-Diamino-3-methyl-1-phenyl-pyrazol, 4,5-Diamino-1-methyl-3-phenylpyrazol, 4-Amino-1,3-dimethyl-5-hydrazino-pyrazol, 1-Benzyl-4,5-diamino-3-methylpyrazol, 4,5-Diamino-3-tert.-butyl-1-methyl-pyrazol, 4,5-Diamino-1-tert.-butyl-3-methylpyrazol, 4,5-Diamino-l-(ß-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethyl-pyrazol, 3,4,5-Triaminopyrazol, 1-Methyl-3,4,5-triaminopyrazol, 3,5-Diamino-1-methyl-4-methylaminopyrazol und 3,5-Diamino-4-(ß-hydroxyethyl)-amino-1-methylpyrazol.

Bevorzugte Pyrazol-Pyrimidin-Derivate sind insbesondere die Derivate des Pyrazol-[1,5-a]-pyrimidin der folgenden Formel (VI) und dessen tautomeren Formen, sofern ein tautomeres Gleichgewicht besteht: 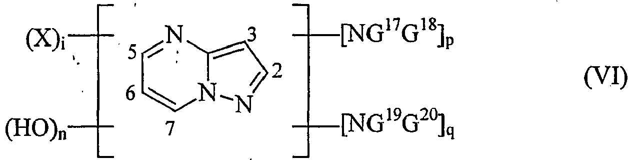 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂-bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
   mit der Maßgabe, daß
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (VI) verwendeten Substituenten sind erfindungsgemäß analog zu den obigen Ausführungen definiert.

Wenn das Pyrazol-[1,5-a]-pyrimidin der obenstehenden Formel (VI) eine Hydroxygruppe an einer der Positionen 2, 5 oder 7 des Ringsystems enthält, besteht ein tautomeres Gleichgewicht, das zum Beispiel im folgenden Schema dargestellt wird:

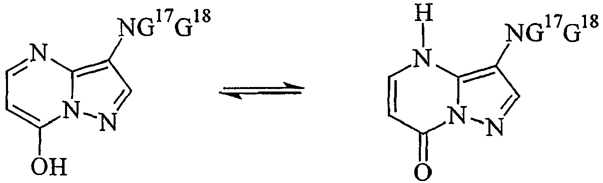

Unter den Pyrazol-[1,5-a]-pyrimidinen der obenstehenden Formel (VI) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)-amino]-ethanol;
- 2-[(7-Aminopyrazol-[1,5-a]-pyrimidin-3-yl)-(2-hydroxy-ethyl)-amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomers Gleichgewicht vorhanden ist. ,

Die Pyrazol-[1,5-a]-pyrimidine der obenstehenden Formel (VI) können wie in der Literatur beschrieben durch Zyklisierung ausgehend von einem Aminopyrazol oder von Hydrazin hergestellt werden.

In einer weiteren bevorzugten Ausführungsform enthalten die erfindungsgemäßen Oxidationsfärbemittel weiterhin mindestens ein Farbstoffvorprodukt, ausgewählt aus der Gruppe der Kupplerkomponenten.

Als Oxidationsfarbstoffvorprodukte vom Kuppler-Typ werden in der Regel m-Phenylendiaminderivate, Naphthole, Resorcin und Resorcinderivate, Pyrazolone und m-Aminophenolderivate verwendet. Beispiele für solche Kupplerkomponenten sind
- m-Aminophenol und dessen Derivate wie beispielsweise 5-Amino-2-methylphenol, 5-(3-Hydroxypropylamino)-2-methylphenol, 3-Amino-2-chlor-6-methylphenol, 2-Hydroxy-4-aminophenoxyethanol, 2,6-Dimethyl-3-aminophenol, 3-Trifluoro-acetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethyl-amino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-amino-benzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamirio-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Methylendioxybenzolderivate wie beispielsweise 1-Hydroxy-3,4-methylen-dioxybenzol, 1-Amino-3,4-methylendioxybenzol und 1-(2'-Hydroxyethyl)-amino-3,4-methylendioxybenzol,

In einer erfindungsgemäß ganz besonders bevorzugten Ausführungsform wird die Wirkstoffkombination des erfindungsgemäßen Oxidationshaarfärbemittels mit Entwickler- und Kupplerkomponenten kombiniert, die dafür bekannt sind, dass sie nur ein sehr geringes oder kein allergenes Potential aufweisen. Bevorzugte Beispiele für solche Entwicklerkomponenten sind 3-Methyl-4-aminophenol, 1-(2'-Hydroxyethyl)-2,5-diaminobenzol, Bis-(5-amino-2-hydroxyphenyl)methan und Tertraaminopyrimidin. Bevorzugte Beispiele für solche Kupplerkomponenten sind 4-Chlorresorcin, 2-Aminophenol, 1,5-Dihydroxynaphthalin, 2,7-Dihydroxynaphthalin, 5-Amino-2-methylphenol, 1-Phenyl-3-methyl-pyrazol-5-on, 2,4-Diaminophenoxyethanol, 2-Methylresorcin, 2-Amino-5-methylphenol, 3,4-Methylendioxyphenol, 2-Amino-4-(2'-hydroxyethylamino)anisol, 3-Amino-2-chlor-6-methylphenol, 1-(2'-Hydroxyethylamino)-3,4-methylendioxybenzol, 3,4-Methylendioxyanilin, 5-Amino-4-chlor-2-methylphenol, 3-Amino-2-methylamino-6-methoxypyridin, 2-Amino-3-hydroxypyridin, 2,6-Bis-(2'hydroxyethyl)aminotoluol, 4-Hydroxy-2,5,6-triamino-pyrimidin, 1,10-Bis-(2,5-diaminophenyl)-1,4,7,10-tetraoxadecan, sowie die physiologisch verträglichen Salze dieser Verbindungen. '

In einer weiteren erfindungsgemäß bevorzugten Ausführungsform weist das Oxidationshaarfärbemittel einen pH-Wert im Bereich von 6 bis 12, insbesondere von 8,5 bis 11 auf.

Eine weiterhin erfindungsgemäß bevorzugte Ausführungsform umfasst Oxidationshaarfärbemittel, die als Konservierungsmittel die Salze der Benzoesäure und der Salicylsäure enthalten.

Zur weiteren Nuancierung können den erfindungsgemäßen Oxidationshaarfärbemitteln weiterhin direktziehende Farbstoffe zugesetzt werden.

Direktziehende Farbstoffe sind üblicherweise Nitrophenylendiamine, Nitroaminophenole, Azofarbstoffe, Anthrachinone oder Indophenole. Besonders geeignete direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Orange 1, HC Red 1, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(β-hydroxyethyl)-amino-2-nitrobenzol, 4-Amino-2-nitrodiphenylamin-2'-carbonsäure, 6-Nitro-1,2,3,4-tetra-hydrochinoxalin, Hydroxyethyl-2-nitro-toluidin, Pikraminsäure, 2-Amino-6-chloro-4-nitrophenol, 4-Ethylamino-3-nitrobenzoesäure, 2-Chloro-6-ethylamino-1-hydroxy-4-nitrobenzol, 4-(2'-Hydroxyethylamino)-3-nitrophenol und 6-Nitro-1,2,3,4-tetrahydrochinoxalin.

In der Natur vorkommende direktziehende Farbstoffe sind beispielsweise die in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthaltenen färbenden Verbindungen.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Als Vorstufen naturanaloger Farbstoffe werden beispielsweise Indole und Indoline sowie deren physiologisch verträgliche Salze verwendet. Bevorzugt werden solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. Besonders vorteilhafte Eigenschaften haben 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin sowie 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxy-indolin und insbesondere das 5,6-Dihydroxyindolin sowie N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden.

Bei der Verwendung von Farbstoff-Vorstufen vom Indolin- oder Indol-Typ kann es bevorzugt sein, diese zusammen mit mindestens einer Aminosäure und/oder mindestens einem Oligopeptid einzusetzen. Bevorzugte Aminosäuren sind Aminocarbonsäuren, insbesondere α-Aminocarbonsäuren und ω-Aminocarbonsäuren. Unter den α-Aminocarbonsäuren sind wiederum Arginin, Lysin, Ornithin und Histidin besonders bevorzugt. Eine ganz besonders bevorzugte Aminosäure ist Arginin, insbesondere in freier Form, aber auch als Hydrochlorid eingesetzt.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 12, eingestellt. Zu diesem Zweck enthalten die Färbemittel Alkalisierungsmittel, üblicherweise Alkali- oder Erdalkalihydroxide, Ammoniak oder organische Amine. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Erfolgt die Ausbildung der eigentlichen Haarfarben im Rahmen eines oxidativen Prozesses, so können übliche Oxidationsmittel, wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel, oder auch Enzyme die Elektronen aus geeigneten Entwicklerkomponenten (Reduktionsmittel) auf Luftsauerstoff übertragen. Bevorzugt sind dabei Oxidasen wie Tyrosinase, Ascorbatoxidase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Zweckmäßigerweise wird die Zubereitung des Oxidationsmittels dann unmittelbar vor dem Färben der Haare mit der Zubereitung mit den Farbstoffvorprodukten vermischt.

Weiterhin kann die Ausbildung der Färbung dadurch unterstützt und gesteigert werden, daß dem Mittel bestimmte Metallionen zugesetzt werden. Solche Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflußt werden.

Die erfindungsgemäßen Oxidationshaarfärbemittel enthalten Farbstoffvorprodukte bevorzugt in einem geeigneten wäßrigen, alkoholischen oder wäßrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Farbstoffvorprodukte in eine pulverförmige oder auch Tabletten-förmige Formulierung zu integrieren.

Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die erfindungsgemäßen Mittel können weiterhin alle für solche Zubereitungen bekannten Wirk-, Zusatz- und Hilfsstoffe enthalten. In vielen Fällen enthalten diese Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa. 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind weitere Alkylpolyglykoside der allgemeinen Formel R'O-(Z)ₓ. Diese Verbindungen sind durch die folgenden Parameter gekennzeichnet.

Der Alkylrest R' enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R'
- im wesentlichen aus C₈- und C₁₀-Alkylgruppen,
- im wesentlichen aus C₁₂- und C₁₄-Alkylgruppen,
- im wesentlichen aus C₈-C₁₆-Alkylgruppen oder
- im wesentlichen aus C₁₂-C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethyl-ammonium-glycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche, oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyl-dimethylammoniumchloride und Trialkylmethylammoniumchloride, z. B. Cetyl-trimethylammoniumchlorid, Stearyltrimethylammoniumchlorid, Distearyldimethylammoniumchlorid, Lauryldimethylammoniumchlorid, Lauryldimethylbenzylammonium-chlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxy-ethyl)dimethylammonium-chlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyldimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, - hydroxide oder -alkoholate als Katalysatoren verwendet werden. Die Verwendung von Produkten mit eingeengter Homologenverteilung kann bevorzugt sein.

Weiterhin können die erfindungsgemäßen Mittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.

Bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat^{®}100 (Poly(dimethyldiallylammoniumchlorid)), Merquat^{®}550 (Dimethyldiallylammoniumchlorid-Acrylamid-Copolymer) und Merquat^{®} 280 (Dimethyldiallyl-ammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{®}734 und Gafquat^{®}755 im Handel erhältlich.
- Vinylpyrrolidon-Methoimidazoliniumchlorid-Copolymere, wie sie unter der Bezeichnung Luviquat^{®} angeboten werden. quaternierter Polyvinylalkohol
   sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquaternium-22.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Coming; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyltrimethylammoniumchlorid/Acrylat-Copolymere und Octylacrylamid/Methylmethacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöl, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein weiterer Gegenstand der Erfindung ist ein Verfahren zur oxidativen Färbung von Keratinfasern, insbesondere Haaren, unter Einsatz eines erfindungsgemäßen Oxidationsfärbemittels. Das eigentliche Haarfärbemittel wird zweckmäßigerweise unmittelbar vor der Anwendung durch Mischung der Zubereitung des Oxidationsmittels mit der Zubereitung, enthaltend die Farbstoffvorprodukte sowie die erfindungsgemäße Wirkstoffkombination, hergestellt. Das dabei entstehende gebrauchsfertige Haarfärbepräparat sollte bevorzugt einen pH-Wert im Bereich von 6 bis 12, insbesondere in einem Bereich von 8,5 bis 11 aufweisen. Besonders bevorzugt ist die Anwendung der Haarfärbemittel in einem alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die eigentliche oxidative Färbung der Fasern kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel. So können die Enzyme (Enzymklasse 1: Oxidoreduktasen) Elektronen aus geeigneten Entwicklerkomponenten (Reduktionsmittel) auf Luftsauerstoff übertragen. Bevorzugt sind dabei Oxidasen wie Tyrosinase, Ascorbat-Oxidase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Ein weiterer Gegenstand der Erfindung ist die Verwendung des erfindungsgemäßen Oxidationshaarfärbemittels zur schonenden Färbung von keratinischen Fasern, insbesondere von Haaren.

Die folgenden Beispiele sollen den Gegenstand der Erfmdung näher erläutern:

### Beispiele

Der synergistische Wirkungsnachweis von Ectoin mit weiteren Pflegekomponenten erfolgte durch Restrukturierungsuntersuchungen oxidativ und reduktiv geschädigter Haare mittels Thermoanalyse sowohl in wässriger Lösung, als auch in konkreten Anwendungsbeispielen, wie Shampoos oder Haarfärbemitteln.

### Dazu wurden die Haare wie folgt vorbehandelt (geschädigt):

Strähnen der Firma Alkino (0,5 g; Code 6634; europäisches Humanhaar, mittelbraun) wurden einer herkömmlichen Dauerwellbehandlung mit dem Handelsprodukt Poly Lock-Normale Dauerwelle unterzogen. Im Rahmen dieser Dauerwellbehandlung wurden die Fasern in einem ersten Schritt für 40 Minuten bei Raumtemperatur der Reduktionslösung (enthaltend 7,9 Gew.-% Thioglycolsäure) ausgesetzt, mit reinem Wasser gespült und anschließend bei Raumtemperatur für 10 Minuten fixiert (Oxidationslösung, enthaltend 2,6 Gew.-% Wasserstoffperoxid). Nach der oxidativen Behandlung wurden die Fasern gespült und getrocknet.

### 1) Syergistischer Wirkungsnachweis von Ectoin und weiteren Pflegekomponenten aus wässriger Lösung bei einem

### a) pH-Wert von 4

Es wurden wässrige Lösungen von 1 % Ectoin mit verschiedenen anderen Wirkstoffen in 1%-Konzentrationen versetzt und mittels Salzsäure der pH-Wert von 4 eingestellt.

Auf jeweils eine Strähne Alkino 6634 (0,5 g) wurde eine der Lösungen (2 ml) appliziert, für 10 Minuten bei Raumtemperatur dort belassen und anschließend 1,5 Minuten abgespült. Nach Trocknung der Strähne erfolgte die thermoanalytische Messung. Als Vergleich wurden die entsprechenden Lösungen mit den Einzelwirkstoffen mitgemessen.

Es wurden die folgenden Denaturierungstemperaturen detektiert:

| **Denaturierungstemperatur [°C]** | | | | | |
|---|---|---|---|---|---|
| **ohne Behandlung** | **Behandlung mit 1% Ectoin** | **Behandlung mit 1 % Allantoin** | **Behandlung mit 1 % Vitamin C** | **Behandlung mit 1 % Bisabolol** | **Behandlung mit 1% Plantacare 1200** |
| 148.19 | 149.34 | 149.15 | 148.38 | 148.38 | 149.61 |
| Δ | 1,15 | 0,96 | 0,19 | 0,19 | 1.42 |

Daraufhin wurden die Haarsträhnen mit den Lösungen der kombinierten Wirkstoffe Ectoin und Allantoin, Ectoin und Vitamin C, Ectoin und Bisabolol sowie Ectoin und Plantacare (jeweils 1 % des Wirkstoffes) behandelt und die folgenden Denaturierunstemperaturen ermittelt:

| | **Denaturierungstemp.** | **Δ_{erwartet}** | **Δ_{gemessen}** |
|---|---|---|---|
| | **[°C]** | **Δ_{Ectoin} + Δ_{Wirkstoff}** | |
| **Ectoin + Allantoin** | 150.60 | 2.11 | 2.41 |
| **Ectoin + Vitamin C** | 150.56 | 1.34 | 2.37 |
| **Ectoin + Bisabolol** | 149.67 | 1.34 | 1.48 |
| **Ectoin + Plantacare 1200** | 150.29 | 2.57 | 2.10 |

Die Messungen zeigen, dass Ectoin in Kombination mit Allantoin, Vitamin C und Bisabolol zu einem signifikant höheren Pflegeeffekt führt als Ectoin allein oder auch die drei zugesetzten Wirkstoffe einzeln, während der Pflegeeffekt des Ectoins in der Kombination mit Plantacare 1200 unter dem zu erwartenden Wert liegt.

### a) pH-Wert von 9

Die Vorbehandlung sowie die Untersuchungen der Haarsträhnen erfolgte wie unter Punkt a) bereits beschrieben; der pH-Wert von 9 wurde durch Zugabe von Monoethanolamin eingestellt.

Es wurden die folgenden Ergebnisse ermittelt:

| **Denaturierungstemperatur [°C]** | | | |
|---|---|---|---|
| **ohne Behandlung** | **Behandlung mit 1% Ectoin** | **Behandlung mit 1 % Allantoin** | **Behandlung mit 1% Plantacare 1200** |
| 145.27 | 147.02 | 145.77 | 145.99 |
| Δ | 1,75 | 0.5 | 0.72 |

Daraufhin wurden die Haarsträhnen mit den Lösungen der kombinierten Wirkstoffe Ectoin und Allantoin sowie Ectoin und Plantacare (jeweils 1 % des Wirkstoffes) behandelt und die folgenden Denaturierunstemperaturen ermittelt:

| | **Denaturierungstemp.** | **Δ_{erwartet}** | **Δ_{gemessen}** |
|---|---|---|---|
| | **[°C]** | **Δ_{Ectoin}** + **Δ_{Wirkstoff}** | |
| **Ectoin + Allantoin** | 148.17 | 2.25 | 2.90 |
| **Ectoin + Plantacare 1200** | 148.34 | 2.47 | 3.07 |

Die Messungen zeigen, dass die Pflegeleistung der Kombination aus Ectoin und Allantoin bei einem alkalischen pH-Wert noch höher ist als im sauren Bereich und dass auch die Kombination von Ectoin und Plantacare 1200 im alkalischen Milieu eine signifikante Pflegeleistung aufweist, die im sauren Milieu nicht beobachtet werden konnte.

### 2) Syergistischer Wirkungsnachweis von Ectoin und weiteren Pflegekomponenten in einer

### a) Shampoorezeptur

Nach der Vorbehandlung wie unter Punkt 1) beschrieben wurde ein Standard-Shampoo (Provitamin B5-Schauma mit einem Gehalt an 0,2% Panthenol) mit 1% Ectoin versetzt. Zusätzlich wurde in weiteren Rezepturen jeweils ein weiterer Pflegestoff zugesetzt. Es wurden die Alkino 6634 Strähnen (0,5 g) für 3 Minuten bei Raumtemperatur mit diesen Formulierungen (2 g) behandelt und anschließend abgespült und abgetrocknet.

Die folgenden Ergebnisse wurden erzielt:

| **Denaturierungstemperatur [°C]** | | | | |
|---|---|---|---|---|
| **ohne Behandl.** | **Behandlung mit Schauma-Provitamin B5-Shampoo** | **Behandlung mit Schauma-Provitamin B5-Shampoo und 1% Ectoin** | **Behandlung mit Schauma-Provitamin B5-Shampoo und 1% Allantoin** | **Behandlung mit mit Schauma-Provitamin B5-Shampoo und 1% Plantacare 1200** |
| 147.14 | 147.69 | 148.64 | 148.22 | 148.01 |
| Δ | 0.55 | 0.95 | 0.53 | 0.32 |

Daraufhin wurden die Haarsträhnen mit dem Provitamin B5-Shampoo, enthaltend zusätzlich die kombinierten Wirkstoffe Ectoin und Allantoin sowie Ectoin und Plantacare (jeweils 1 % des Wirkstoffes) behandelt und die folgenden Denaturierunstemperaturen ermittelt:

| | **Denaturierungstemp.** | **Δ_{erwartet}** | **Δ_{gemessen}** |
|---|---|---|---|
| | **[°C]** | **Δ_{Shampoo} + Δ_{Ectoin} + Δ_{Wirkstoff}** | |
| **Ectoin + Allantoin** | 150.19 | 2.03 | 2.50 |
| **Ectoin + Plantacare 1200** | 149.77 | 1.82 | 2.63 |

Die Werte zeigen, dass das Shampoo ohne Ectoin aber mit einem Gehalt an Provitamin B5 bereits eine Tendenz zur Restrukturierung zeigt, durch Zusatz von Ectoin eine gesteigerte Wirkung erreicht wird; durch Kombination der Stoffe allerdings ein signifikant höherer (synergistischer) Effekt erzielt wird.

### b) Oxidationshaarfarbe

Nach der Vorbehandlung wie unter Punkt 1) beschrieben wurde die Rezeptur Poly Diadem Hellblond (ohne Plantacare) mit 1% Ectoin sowie mit jeweils 1 % eines anderen Wirkstoffs versetzt. Als Referenz wurde die Marktrezeptur Poly Diadem Hellblond, die bereits 1% Plantacare 1200 enthält, untersucht. Alkino 6634 Haarsträhnen (0,5 g) wurden für 30 Minuten bei Raumtemperatur mit diesen Rezepturen behandelt und anschließend gespült und getrocknet. Die darauffolgende DSC-Messung ergab die folgenden Ergebnisse:

| **Denaturierungstemperatur [°C]** | | | |
|---|---|---|---|
| **Behandlung mit Poly Diadem Hellblond ohne Plantacare (oxidative Schädigung der Haare)** | **Behandlung mit Poly Diadem Hellblond mit Plantacare (Marktrezeptur)** | **Behandlung mit Poly Diadem Hellblond ohne Plantacare und 1% Ectoin** | **Behandlung mit Poly Diadem Hellblond ohne Plantacare und 1% Allantoin** |
| 142.51 | 143.26 | 143.30 | 143.43 |
| Δ | 0.75 | 0.79 | 0.92 |

Daraufhin wurden die Haarsträhnen mit der Poly Diadem Hellblond-Rezeptur, enthaltend die kombinierten Wirkstoffe Ectoin und Allantoin sowie Ectoin und Plantacare (jeweils 1 % des Wirkstoffes) behandelt, und die folgenden Denaturierunstemperaturen ermittelt:

| | **Denaturierungstemp.** | **Δ_{erwartet}** | **Δ_{gemessen}** |
|---|---|---|---|
| | **[°C]** | **Δ_{Ectoin} + Δ_{Wirkstoff}** | |
| **Ectoin + Allantoin** | 144.97 | 1.71 | 2.46 |
| **Ectoin + Plantacare 1200** | 144.15 | 1.54 | 1.64 |

Zusätzlich wurde die Marktrezeptur Poly Diadem Hellblond, die bereits Plantacare enthält, mit der Wirkstoffkombination Ectoin und Allantoin versetzt. Aus der ersten Tabelle wäre ein Δ von 2.46 zu erwarten - es wurde jedoch eine Denaturierungstemperatur von 145.10°C ermittelt, was einem Δ von 2.59 entspricht!

Die Gesamtheit der Ergebnisse belegt, dass die erfindungsgemäßen Wirkstoffkombinationen in synergistischer Weise geschädigte Haare restrukturieren und eine Verringerung der oxidativen Schädigung bei der oxidativen Haarfärbung bewirken.

## Patentansprüche

1. Oxidationshaarfärbemittel, **dadurch gekennzeichnet, dass** es in einem zum Färben geeigneten Medium eine Wirkstoffkombination, bestehend aus mindestens einer Verbindung der allgemeinen Formel (1) oder (II) 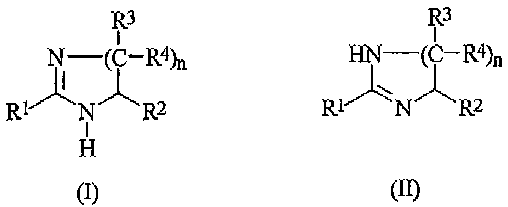 und/oder eines physiologisch verträglichen Salzes und/oder einer isomeren oder stereoisomeren Form dieser Verbindungen, in denen
- R¹ steht für ein Wasserstoffatom, einen verzweigten oder unverzweigten C₁-C₄-Alkylrest oder einen C₂ - C₄-Hydroxyalkylrest,
- R² steht für ein Wasserstoffatom, eine Gruppierung -COOR⁵ oder eine Gruppierung -CO(NH)R⁵, wobei R⁵ für ein Wasserstoffatom, einen C₁ -C₄-Alkylrest, einen Aminosäurerest, einen Dipeptid- oder einen Tripeptidrest stehen kann,
- R³ und R⁴ stehen unabhängig voneinander für ein Wasserstoffatom, einen C₁
- C₄-Alkylrest oder einer der beiden Reste steht für eine Hydroxygruppe und
- n steht für eine ganze Zahl von 1 bis 3,
und mindestens einer weiteren Komponente, ausgewählt aus der Gruppe, die gebildet wird aus Alkylpolyglucosiden, Allantoin sowie den physiologisch verträglichen Derivaten dieser Verbindungen, sowie mindestens ein Farbstoffvorprodukt, ausgewählt aus der Gruppe der Entwicklerkomponenten, enthält.

2. Oxidationsmittel nach Anspruch 1, **dadurch gekennzeichnet, dass** das Gewichtsverhältnis der Verbindung nach Formel (I) zu den Alkylpolyglucosiden 1:1 bis 1:3 und/oder das Gewichtsverhältnis der Verbindung nach Formel (I) zu den Allantoin 2:1 bis 1:2 beträgt.

3. Oxidationsmittel nach einem der Ansprüche 1 oder 2, **dadurch gekennzeichnet, dass** die Verbindung gemäß Formel (I) oder (II) Ectoin oder eines seiner physiologisch verträglichen Salze ist.

4. Oxidationsmittel nach einem der Ansprüche 1 bis 3, **dadurch gekennzeichnet, dass** der Mengenanteil des Ectoins oder dessen physiologisch verträglichen Salzen 0,01 bis 5 Gew.-%, bezogen auf das Gesamtgewicht des Haarbehandlungsmittels, beträgt.

5. Oxidationshaarfärbemittel nach einem der Ansprüche 1 bis 4, **dadurch gekennzeichnet, dass** es als Farbstoffvorprodukt mindestens ein Pyrimidinderivat und/oder ein physiologisch verträgliches Salz eines Pyrimidinderivats enthält.

6. Oxidationshaarfärbemittel nach einem der Ansprüche 1 bis 5, **dadurch gekennzeichnet, dass** es 2,4,5,6-Tetraaminopyrimidin oder ein physiologisch verträgliches Salz dieser Verbindung enthält.

7. Oxidationshaarfärbemittel nach Anspruch 1, **dadurch gekennzeichnet, dass** es 1-(2'-Hydroxyethyl)-2,5-diaminobenzol oder ein physiologisch verträgliches Salz dieser Verbindung enthält.

8. Oxidationshaarfärbemittel nach Anspruch 1, **dadurch gekennzeichnet, dass** es 3-Methyl-4-aminophenol oder ein physiologisch verträgliches Salz dieser Verbindung enthält.

9. Oxidationshaarfärbemittel nach Anspruch 1, **dadurch gekennzeichnet, dass** es Bis-(2-hydroxy-5-aminophenyl)-methan oder ein physiologisch verträgliches Salz dieser Verbindung enthält.

10. Oxidationshaarfärbemittel nach einem der Ansprüche 1 bis 9, **dadurch gekennzeichnet, dass** es weiterhin mindestens eine Kupplerkomponente enthält.

11. Oxidationsfärbemittel nach Anspruch 10, **dadurch gekennzeichnet, dass** die Kupplerkomponente ausgewählt ist aus 4-Chlorresorcin, 2-Aminophenol, 1,5-Dihydroxynaphthalin, 2,7-Dihydroxynaphthalin, 5-Amino-2-methylphenol, 1-Phenyl-3-methyl-pyrazol-5-on, 2,4-Diaminophenoxyethanol, 2-Methylresorcin, 2-Amino-5-methylphenol, 3,4-Methylendioxyphenol, 2-Amino-4-(2'-hydroxyethylamino)anisol, 3-Amino-2-chlor-6-methylphenol, 1-(2'-Hydroxyethylamino)-3,4-methylendioxybenzol, 3,4-Methylendioxyanilin,
5-Amino-4-chlor-2-methylphenol, 3-Amino-2-methylamino-6- methoxypyridin, 2-Amino-3-hydroxypyridin, 2,6-Bis-(2'hydroxyethyl)aminotoluol, 4-Hydroxy-2,5,6-triamino-pyrimidin, 1,10-Bis-(2,5-diaminophenyl)-1,4,7,10-tetraoxadecan, sowie die physiologisch verträglichen Salze dieser Verbindungen.

12. Oxidationshaarfärbemittel nach einem der Ansprüche 1 bis 11, **dadurch gekennzeichnet, dass** es weiterhin mindestens einen direktziehenden Farbstoff enthält, der ausgewählt ist aus der Gruppe die gebildet wird aus HC Yellow 4, HC Orange 1, HC Red 1, 4- ('-Hydroxyethylamino)-3-nitrophenol, 4-Amino-2-nitro-diphenylamin-2'-carbonsäure, 2-Amino-6-chlor-4-nitrophenol, 2-Ethylamino-6-chlor-4-nitrophenol und 6-Nitro-1,2,3,4-tetrahydrochinoxalin.

13. Oxidationshaarfärbemittel nach einem der Ansprüche 1 bis 12, **dadurch gekennzeichnet, dass** es weiterhin mindestens einen naturanalogen Farbstoff aus der Gruppe der Indole und Indoline enthält.

14. Verfahren zur oxidativen Haarfärbung, **dadurch gekennzeichnet, dass** ein Mittel nach einem der Ansprüche 1 bis 13 auf die Haare aufgebracht und nach einer Einwirkungszeit ausgespült oder mit einem Shampoo ausgewaschen wird.

15. Verwendung eines Mittels nach einem der Ansprüche 1 bis 13 zur oxidativen Haarfärbung.

## Claims

1. An oxidising hair colouring agent **characterised in that** it contains, in a medium suitable for colouring, a combination of active ingredients consisting of at least one compound of the general formula (I) or (II) 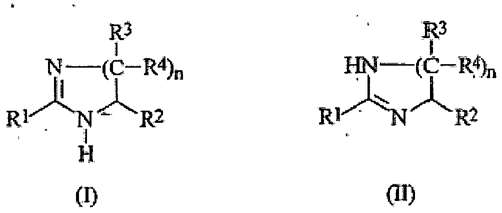 and/or of a physiologically acceptable salt and/or of an isomeric or stereoisomeric form of those compounds, in which
- R¹ denotes a hydrogen atom, a branched or unbranched C₁ to C₄ alkyl residue or a C₂ to C₄ hydroxyalkyl residue,
- R² denotes a hydrogen atom, a -COOR⁵ group or a -CO(NH)R⁵ group, wherein R⁵ can denote a hydrogen atom, a C₁ to C₄ alkyl residue, an amino acid residue, a dipeptide residue or a tripeptide residue,
- R³ and R⁴ mutually independently denote a hydrogen atom or a C₁ to C₄ alkyl residue, or one of the two residues denotes a hydroxy group and
- n denotes an integer from 1 to 3,
and at least one further component selected from the group constituted by alkylpolyglucosides, allantoin and the physiologically acceptable derivatives of those compounds, as well as at least one dye precursor selected from the group of the developer components.

2. The oxidising agent according to claim 1, **characterised in that** the ratio by weight of the compound of formula (I) to the alkylpolyglucosides is 1:1 to 1:3 and/or the ratio by weight of the compound of formula ((I) to allantoin is 2:1 to 1:2.

3. The oxidising agent according to one of claims 1 or 2, **characterised in that** the compound according to formula (I) or (II) is ectoin or one of its physiologically acceptable salts.

4. The oxidising agent according to one of claims 1 to 3, **characterised in that** the quantitative proportion of ectoin or of its physiologically acceptable salts is 0.01 to 5 wt% based on the total weight of the hair treatment agent.

5. The oxidising hair colouring agent according to one of claims 1 to 4, **characterised in that** it contains as a dye precursor at least one pyrimidine derivative and/or a physiologically acceptable salt of a pyrimidine derivative.

6. The oxidising hair colouring agent according to one of claims 1 to 5, **characterised in that** it contains 2,4,5,6-tetraaminopyrimidine or a physiologically acceptable salt of that compound.

7. The oxidising hair colouring agent according to claim 1, **characterised in that** it contains 1-(2'-hydroxyethyl)-2,5-diaminobenzene or a physiologically acceptable salt of that compound.

8. The oxidising hair colouring agent according to claim 1, **characterised in that** it contains 3-methyl-4-aminophenol or a physiologically acceptable salt of that compound.

9. The oxidising hair colouring agent according to claim 1, **characterised in that** it contains bis-(2-hydroxy-5-aminophenyl)methane or a physiologically acceptable salt of that compound.

10. The oxidising hair colouring agent according to one of claims 1 to 9, **characterised in that** it furthermore contains at least one coupler component.

11. The oxidising hair colouring agent according to claim 10, **characterised in that** the coupler component is selected from 4-chlororesorcinol, 2-aminophenol, 1,5-dihydroxynaphthalene, 2,7-dihydroxynaphthalene, 5-amino-2-methylphenol, 1-phenyl-3-methylpyrazol-5-one, 2-4-diaminophenoxyethanol, 2-methylresorcinol, 2-amino-5-methylphenol, 3,4-methylenedioxyphenol, 2-amino-4-(2'-hydroxyethylamino)anisole, 3-amino-2-chloro-6-methylphenol, 1-(2'-hydroxyethylamino)-3,4-methylenedioxybenzene, 3,4-methylenedioxyaniline, 5-amino-4-chloro-2-methylphenol, 3-amino-2-methylamino-6-methoxypyridine, 2-amino-3-hydroxypyridine, 2,6-bis-(2'-hydroxyethyl)aminotoluene, 4-hydroxy-2,5,6-triaminopyrimidine, 1,10-bis-(2,5-diaminophenyl)-1,4,7,10-tetraoxadecane, as well as the physiologically acceptable salts of those compounds.

12. The oxidising hair colouring agent according to one of claims 1 to 11, **characterised in that** it furthermore contains at least one substantive dye that is selected from the group constituted by HC Yellow 4, HC Orange 1, HC Red 1, 4-(2'-hydroxyethylamino)-3-nitrophenol, 4-amino-2-nitro-diphenylamino-2'-carboxylic acid, 2-amino-6-chloro-4-nitrophenol, 2-ethylamino-6-chloro-4-nitrophenol and 6-nitro-1,2,3,4-tetrahydroquinoxaline.

13. The oxidising hair colouring agent according to one of claims 1 to 12, **characterised in that** it furthermore contains at least one bioanalogous dye from the group of indoles and indolines.

14. A method for oxidative colouring of hair, **characterised in that** an agent according to one of claims 1 to 13 is applied on the hair and, after a contact time, is rinsed out or is washed out with a shampoo.

15. Use of an agent according one of claims 1 to 13 for oxidising hair colouring.

## Revendications

1. Agent de teinture des cheveux par oxydation, **caractérisé en ce qu'**il contient, dans un milieu approprié pour la teinture, une combinaison de substances actives, constituée par au moins un composé de formule générale (I) ou (II) 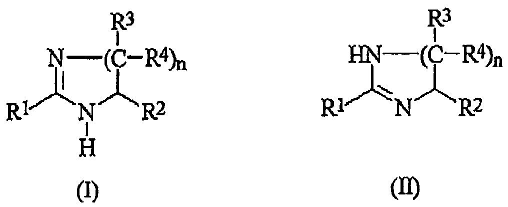 et/ou un sel physiologiquement acceptable et/ou une forme isomère ou stéréo-isomère de ces composés, dans lesquelles
- R¹ représente un atome d'hydrogène, un radical C₁-C₄-alkyle ramifié ou linéaire ou un radical C₂-C₄-hydroxyalkyle,
- R² représente un atome d'hydrogène, un groupe -COOR⁵ ou un groupe -CO(NH)R⁵, R⁵ pouvant représenter un atome d'hydrogène, un radical C₁-C₄-alkyle, un radical acide aminé, un radical dipeptide ou un radical tripeptide,
- R³ et R⁴ représentent, indépendamment l'un de l'autre, un atome d'hydrogène, un radical C₁-C₄-alkyle ou un des deux radicaux représente un groupe hydroxy et
- n représente un nombre entier de 1 à 3,
et au moins un autre composant, choisi dans le groupe formé par les alkylpolyglucosides, l'allantoïne ainsi que les dérivés physiologiquement acceptables de ces composés, ainsi qu'au moins un précurseur de colorant, choisi dans le groupe des composants développeurs.

2. Agent d'oxydation selon la revendication 1, **caractérisé en ce que** le rapport pondéral du composé selon la formule (I) aux alkylpolyglucosides vaut 1:1 à 1:3 et/ou le rapport pondéral du composé selon la formule (I) à l'allantoïne vaut 2:1 à 1:2.

3. Agent d'oxydation selon l'une quelconque des revendications 1 ou 2, **caractérisé en ce que** le composé selon la formule (I) ou (II) est l'ectoïne ou un de ses sels physiologiquement acceptables.

4. Agent d'oxydation selon l'une quelconque des revendications 1 à 3, **caractérisé en ce que** la quantité d'ectoïne ou de ses sels physiologiquement acceptables est de 0,01 à 5 % en poids, par rapport au poids total de l'agent de traitement des cheveux.

5. Agent de teinture par oxydation selon l'une quelconque des revendications 1 à 4, **caractérisé en ce qu'**il contient, comme précurseur de colorant, au moins un dérivé de pyrimidine et/ou un sel physiologiquement acceptable d'un dérivé de pyrimidine.

6. Agent de teinture par oxydation des cheveux selon l'une quelconque des revendications 1 à 5, **caractérisé en ce qu'**il contient de la 2,4,5,6-tétraaminopyrimidine ou un sel physiologiquement acceptable de ce composé.

7. Agent de teinture des cheveux par oxydation selon la revendication 1, **caractérisé en ce qu'**il contient du 1-(2'-hydroxyéthyl)-2,5-diaminobenzène ou un sel physiologiquement acceptable de ce composé.

8. Agent de teinture des cheveux par oxydation selon la revendication 1, **caractérisé en ce qu'**il contient du 3-méthyl-4-aminophénol ou un sel physiologiquement acceptable de ce composé.

9. Agent de teinture des cheveux par oxydation selon la revendication 1, **caractérisé en ce qu'**il contient du bis-(2-hydroxy-5-aminophényl)-méthane ou un sel physiologiquement acceptable de ce composé.

10. Agent de teinture par oxydation des cheveux selon l'une quelconque des revendications 1 à 9, **caractérisé en ce qu'**il contient en outre au moins un composant de couplage.

11. Agent de teinture par oxydation selon la revendication 10, **caractérisé en ce que** le composant de couplage est choisi parmi le 4-chlororésorcinol, le 2-aminophénol, le 1,5-dihydroxynaphtalène, le 2,7-dihydroxynaphtalène, le 5-amino-2-méthylphénol, la 1-phényl-3-méthylpyrazol-5-one, le 2,4-diaminophénoxyéthanol, le 2-méthylrésorcinol, le 2-amino-5-méthylphénol, le 3,4-méthylènedioxyphénol, le 2-amino-4-(2'-hydroxyéthylamino)anisol, le 3-amino-2-chloro-6-méthylphénol, le 1-(2'-hydroxyéthylamino)-3,4-méthylènedioxybenzène, la 3,4-méthylènedioxyaniline, le 5-amino-4-chloro-2-méthylphénol, la 3-amino-2-méthylamino-6-méthoxypyridine, la 2-amino-3-hydroxypyridine, le 2,6-bis-(2'hydroxyéthyl)aminotoluène, la 4-hydroxy-2,5,6-triaminopyrimidine, le 1,10-bis-(2,5-diaminophényl)-1,4,7,10-tétraoxadécane, ainsi que les sels physiologiquement acceptables de ces composés.

12. Agent de teinture par oxydation des cheveux selon l'une quelconque des revendications 1 à 11, **caractérisé en ce qu'**il contient en outre au moins un colorant montant directement sur les fibres, qui est choisi dans le groupe formé par le HC Yellow 4, le HC Orange 1, le HC Red 1, le 4-(2'-hydroxyéthylamino)-3-nitrophénol, l'acide 4-amino-2-nitrodiphénylamine-2'-carboxylique, le 2-amino-6-chloro-4-nitrophénol, le 2-éthylamino-6-chloro-4-nitrophénol et la 6-nitro-1,2,3,4-tétrahydroquinoxaline.

13. Agent de teinture par oxydation des cheveux selon l'une quelconque des revendications 1 à 12, **caractérisé en ce qu'**il contient en outre au moins un colorant analogue à un colorant naturel du groupe des indoles et indolines.

14. Procédé pour la teinture par oxydation des cheveux, **caractérisé en ce qu'**on applique un agent selon l'une quelconque des revendications 1 à 13 sur les cheveux et on l'élimine après un temps d'action par rinçage ou par lavage avec un shampooing.

15. Utilisation d'un agent selon l'une quelconque des revendications 1 à 13 pour la teinture par oxydation des cheveux.
